# EUROPEAN PATENT APPLICATION

(11) **EP 4 424 758 A1**
(43) Date of publication of application: **04.09.2024**
(21) Application number: 22886766.9
(22) Date of filing: 17.10.2022
(51) Int. Cl.: C08J 11/10, C07D 201/12

(54) **METHOD OF PRODUCING THERMOPLASTIC RESIN**

(30) Priority: 29.10.2021 JP 2021178293
(71) Applicant: Toray Industries, Inc., Tokyo 103-8666 (JP)
(72) Inventor: YAMASHITA, Kohei, Nagoya-shi, Aichi 455-8502 (JP); TAKAHASHI, Akihiro, Nagoya-shi, Aichi 455-8502 (JP); KATO, Masashi, Tokai-shi, Aichi 476-8567 (JP); NISHIMURA, Mihoko, Nagoya-shi, Aichi 455-8502 (JP); KATO, Koya, Nagoya-shi, Aichi 455-8502 (JP)
(74) Representative: Mewburn Ellis LLP
(86) International application number: PCT/JP2022/038587
(87) International publication number: WO 2023/074438

(57) **Abstract**

To provide a method for producing a thermoplastic resin using ε-caprolactam obtained by depolymerizing a waste of a polyamide 6 resin composition molded body in an energy saving manner using only a small amount of water or the like. The present invention is a method for producing a thermoplastic resin, including obtaining ε-caprolactam by steps (a) and (b) below using a waste (A) of a resin molded body containing at least polyamide 6 as a raw material, and polymerizing a raw material containing the ε-caprolactam:
(a) a step of adding at least one of water (B) heated to 290°C or higher and 350°C or lower and a polyamide 6 oligomer aqueous solution (B1) heated to 290°C or higher and 350°C or lower to the waste (A) of the resin molded body to make contact with each other; and
(b) a step of separating a reaction mixture obtained in the step (a) into a solid and an aqueous solution containing ε-caprolactam by a solid-liquid separation (I).

## Description

### Field

The present invention relates to a method for producing a thermoplastic resin from a polyamide 6 resin composition molded body waste through ε-caprolactam, the method achieving both resource circulation utilization and reduction in global warming gas emissions, the method including depolymerizing the polyamide 6 resin composition molded body waste using only a small amount of water having a high specific heat capacity and a high vaporization heat, and producing a thermoplastic resin using the obtained ε-caprolactam with high purity.

### Background

In recent years, with the ocean plastic problem as a trigger, an interest in global environmental issues has increased, and there is a growing recognition that construction of a sustainable society is required. Global environmental issues include global warming, resource depletion, water shortage, and the like, and most of them are caused by an increase in resource consumption and global warming gas emissions due to rapid human activities after the industrial revolution. Therefore, in order to construct a sustainable society, technologies related to circulation utilization of fossil resources such as plastics and reduction in global warming gas emissions are increasingly important.

As a plastic recycling technology, a technology for conversion into a pyrolysis oil by thermally decomposing a plastic waste material and collecting a gas, an oil, or the like has attracted attention, and many methods have been proposed. For example, Patent Literature 1 discloses a method for producing a hydrocarbon by a process including thermal decomposition and steam cracking of a waste plastic. These methods have an advantage that mixed waste plastics can be converted into a pyrolysis oil but cracking at a high temperature of 800°C or higher is required in order to convert a pyrolysis oil into a secondary raw material such as a plastic monomer, and further, when, for example, a plastic containing chlorine such as polyvinyl chloride or sulfur such as polyarylene sulfide is mixed in waste plastics, there is a problem of plant corrosion, and when a plastic containing oxygen and nitrogen such as a polyamide is mixed therein, there is a concern about explosion.

As a method for recycling polyamide 6, which is used in a large amount in various fields as a fiber, a film, and an engineering plastic, a method for obtaining ε-caprolactam by blowing superheated steam in the presence of a phosphoric acid catalyst is disclosed (see, for example, Patent Literature 2).

In addition, as a method for depolymerizing polyamide 6 without using a catalyst such as an acid or a base, a method for collecting a lactam by bringing polyamide 6 and superheated water into contact with each other at a temperature of 280°C to 320°C is disclosed (see, for example, Patent Literatures 3 and 4.).

### Citation List

### Patent Literature

Patent Literature 1: JP 2019-533041 A
Patent Literature 2: JP H08-217746 A
Patent Literature 3: JP H10-510280 A
Patent Literature 4: JP H10-510282 A

### Summary

### Technical Problem

The method for collecting ε-caprolactam disclosed in Patent Literature 2 is a high-yield reaction in which the depolymerization yield of polyamide 6 is 80% or more, but requires a long time for the depolymerization reaction. Further, since a large amount of superheated water vapor, which is about 10 times the amount of polyamide 6 fibers, is required, the technology still has a problem in achieving both circulation utilization of fossil resources and reduction in global warming gas emissions. In addition, since this method is a reaction using phosphoric acid as a catalyst, the reaction is susceptible to impurities such as catalyst deactivation due to an additive contained in a plastic or adhering impurities in a waste plastic. In fact, it has been found that when the present inventors have conducted a collection experiment using polyamide 6 containing a potassium salt as a raw material under the same or similar conditions to the method described in Patent Literature 2, the yield significantly decreases. This is considered to be due to deactivation of the phosphoric acid catalytic action by the potassium salt.

Meanwhile, in the methods for collecting ε-caprolactam disclosed in Patent Literatures 3 and 4, only water is used in the depolymerization reaction, and a catalyst such as phosphoric acid is not used, so that there is an advantage that reaction deactivation due to an additive, adhering impurities, or the like does not occur. However, in the disclosed method for collecting ε-caprolactam, water having a specific heat capacity of 4.2 kJ/kg·K and a vaporization heat of 2,250 kJ/kg, which are very high, is used in an amount about 10 times the amount of polyamide 6, which is large, to carry out a reaction for a long time, and thus a large amount of energy is required in the depolymerization reaction and in the collection of ε-caprolactam from a low concentration ε-caprolactam aqueous solution. In addition, even when the amount of water used was simply reduced under the same conditions or similar conditions, the collection rate of ε-caprolactam only decreased. This is considered to be because the thermodynamic equilibrium point of ε-caprolactam generated by depolymerization and a linear oligomer generated by hydrolytic ring-opening of ε-caprolactam moved to the linear oligomer side only by simply reducing the amount of water used.

### Solution to Problem

In order to solve the above problems, the present invention has the following configurations.
1. A method for producing a thermoplastic resin, including obtaining ε-caprolactam by steps (a) and (b) below using a waste (A) of a resin molded body containing at least polyamide 6 as a raw material, and polymerizing a raw material containing the ε-caprolactam:
   (a) a step of adding at least one of water (B) heated to 290°C or higher and 350°C or lower and a polyamide 6 oligomer aqueous solution (B1) heated to 290°C or higher and 350°C or lower to the waste (A) of the resin molded body to make contact with each other; and
   (b) a step of separating a reaction mixture obtained in the step (a) into a solid and an aqueous solution containing ε-caprolactam by a solid-liquid separation (I).
2. The method for producing a thermoplastic resin according to item 1, wherein the step (a) is a step of adding water (B) heated to 290°C or higher and 350°C or lower, or adding water (B) heated to 290°C or higher and 350°C or lower and a polyamide 6 oligomer aqueous solution (B1) heated to 290°C or higher and 350°C or lower, to the waste (A) of the resin molded body to make contact with each other.
3. The method for producing a thermoplastic resin according to item 1 or 2, wherein the solid-liquid separation (I) includes a step (b1) of separating the reaction mixture into a non-melting material and an aqueous solution containing at least ε-caprolactam and a polyamide 6 oligomer by a solid-liquid separation, and a step (b2) of separating a filtrate obtained in the step (b1) into the polyamide 6 oligomer and an ε-caprolactam aqueous solution by a solid-liquid separation.
4. The method for producing a thermoplastic resin according to any one of items 1 to 3, wherein a polyamide 6 oligomer aqueous solution (B1) obtained by mixing the polyamide 6 oligomer separated in either the step (b) or the step (b2) with water, followed by heating to 290°C or higher and 350°C or lower is used in the step (a).
5. The method for producing a thermoplastic resin according to any one of items 1 to 4, wherein the polyamide 6 oligomer aqueous solution (B1) is an extract liquid obtained in a step of extracting a polyamide 6 oligomer with hot water from polyamide 6 which is a product at a time of production of polyamide 6.
6. The method for producing a thermoplastic resin according to any one of items 1 to 5, wherein in the step (a), the contact is made under a condition that a product of X and Y is 2,000 or less when a mass ratio of water to polyamide 6 in the waste (A) of the resin molded body or a mass ratio of water to a sum of polyamide 6 and a polyamide oligomer in the waste (A) of the resin molded body is represented by X : 1 and a reaction temperature is represented by Y°C.

### Advantageous Effects of Invention

The present invention can provide a method for producing a thermoplastic resin using ε-caprolactam with high purity obtained by depolymerizing a waste of a polyamide 6 resin composition molded body in an energy saving manner using only a small amount of water having a high specific heat capacity and high vaporization heat or a polyamide 6 oligomer aqueous solution containing water as a solvent.

### Description of Embodiments

Hereinafter, the present invention will be described in more detail.

### (1) Waste (A) of resin molded body

The present invention is a method for producing a thermoplastic resin, in which ε-caprolactam is obtained using a waste (A) of a resin molded body containing at least polyamide 6 as a raw material; and a raw material containing the ε-caprolactam is polymerized.

The polyamide 6 used in the present invention is a polyamide resin in which 6-aminocaproic acid and/or ε-caprolactam is used as a main raw material. The polyamide 6 may be one obtained by copolymerization with another monomer as long as the object of the present invention is not impaired. Here, the phrase "used as a main raw material" means that a total of 50 mol% or more of a unit derived from 6-aminocaproic acid or a unit derived from ε-caprolactam is contained in a total of 100 mol% of monomer units included in the polyamide resin. A unit derived from 6-aminocaproic acid or a unit derived from ε-caprolactam is more preferably contained in an amount of 70 mol% or more, and still more preferably 90 mol% or more.

Examples of another monomer to be copolymerized include amino acids such as 11-aminoundecanoic acid, 12-aminododecanoic acid, and para-aminomethylbenzoic acid, lactams such as ω-laurolactam, aliphatic diamines such as tetramethylenediamine, pentamethylenediamine, hexamethylenediamine, 2-methylpentamethylenediamine, nonamethylenediamine, decamethylenediamine, undecamethylenediamine, dodecamethylenediamine, 2,2,4-/2,4,4-trimethylhexamethylenediamine, and 5-methylnonamethylenediamine, aromatic diamines such as meta-xylylenediamine and para-xylylenediamine, alicyclic diamines such as 1,3-bis(aminomethyl)cyclohexane, 1,4-bis(aminomethyl)cyclohexane, 1-amino-3-aminomethyl-3,5,5-trimethylcyclohexane, bis(4-aminocyclohexyl)methane, bis(3-methyl-4-aminocyclohexyl)methane, 2,2-bis(4-aminocyclohexyl)propane, 1,4-bis(3-aminopropyl)piperazine, and 1-(2-aminoethyl)piperazine, aliphatic dicarboxylic acids such as adipic acid, suberic acid, azelaic acid, sebacic acid, and dodecanedioic acid, aromatic dicarboxylic acids such as terephthalic acid, isophthalic acid, 2-chloroterephthalic acid, 2-methylterephthalic acid, 5-methylisophthalic acid, 5-sodium sulfoisophthalate, and 2,6-naphthalenedicarboxylic acid, and alicyclic dicarboxylic acids such as 1,4-cyclohexanedicarboxylic acid, 1,3-cyclohexanedicarboxylic acid, 1,2-cyclohexanedicarboxylic acid, and 1,3-cyclopentanedicarboxylic acid. Two or more of these may be copolymerized.

In addition, a polymerization degree regulator, a terminal group regulator or the like may be added to such polyamide 6. Examples of the polymerization degree regulator and the terminal group regulator include acetic acid and benzoic acid.

The polymerization degree of the polyamide 6 of the present invention is not particularly limited, but the relative viscosity measured at 25°C in a 98% concentrated sulfuric acid solution having a resin concentration of 0.01 g/mL is preferably in a range of 1.5 to 5.0. The relative viscosity in such a preferable range can be preferably exemplified because the reaction efficiency with a small amount of water tends to increase.

The polyamide 6 of the present invention may contain a cyclic oligomer represented by the following formula (a). The amount of the cyclic oligomer represented by the following formula (a) contained in the polyamide 6 is not particularly limited, but can be exemplified by preferably 2.0 mass% or less, more preferably 1.8 mass% or less, and still more preferably 1.5 mass% or less. In the cyclic oligomer represented by the following formula (a), m is an integer of 2 to 4. Since the cyclic oligomer represented by the following formula (a) melts and volatilizes to cause line blockage or the like, when the amount of the cyclic oligomer is in the preferable range, there is a tendency that line blockage due to melting and volatilization can be prevented. Note that the cyclic oligomer represented by the following formula (a) in which m is 5 or more is not the focus of the present invention in consideration of the degree of volatilization thereof.

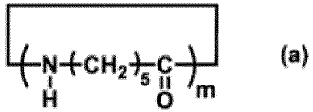

In the present invention, a waste of a resin molded body containing at least polyamide 6 is used as a raw material. The waste of the resin molded body here may be any waste of a resin molded body containing at least polyamide 6. Examples of the waste of the resin molded body containing polyamide 6 include a polyamide 6 product, an industrial waste generated in the process for producing a polyamide 6 product, and a used polyamide 6 product waste. Examples of the polyamide 6 product include fiber structures for clothing such as old clothes, uniforms, sportswear, and inner wear, industrial fiber structures such as curtains, carpets, ropes, nets, belts, and sheets, molded parts for residential building materials, electrical and electronic molded parts, aircraft parts, industrial machine parts, film products, extrusion molded products, in situ polymerized molded products, and RIM molded products. In addition, product scraps, pellet scraps, block scraps, cutting scraps during cutting work, and the like generated in these production steps also serve as waste targets.

The waste (A) of the resin molded body of the present invention may further contain an alkali metal halide as long as the object of the present invention is not impaired. Examples of the alkali metal halide include alkali metal halides such as lithium iodide, sodium iodide, potassium iodide, lithium bromide, sodium bromide, potassium bromide, lithium chloride, sodium chloride, and potassium chloride, and two or more of these can be used in combination. Among them, potassium iodide is preferable from the viewpoint of easy availability, excellent dispersibility in polyamide 6, higher reactivity with radicals, and further improvement of retention stability at a high temperature. Further, these alkali metal halides are more preferably used in combination with a Group 11 metal halide such as copper(I) iodide, copper(I) bromide, or copper(I) chloride because the retention stability at a high temperature is further improved.

Such an alkali metal halide is preferably blended in an amount of 0.01 to 1 part by mass with respect to 100 parts by mass of polyamide 6 in the waste (A) of the resin molded body. By blending the alkali metal halide in such a preferable range, side reactions other than hydrolysis in the present process can be prevented, and the lactam yield tends to increase. The blending amount of the alkali metal halide is more preferably 0.02 to 0.5 parts by mass, and still more preferably 0.03 to 0.4 parts by mass.

The waste (A) of the resin molded body of the present invention may contain a fibrous filling material. The fibrous filling material here may be any filling material having a fibrous shape. Specific examples thereof include glass fibers, polyacrylonitrile (PAN)-based or pitch-based carbon fibers, metal fibers such as stainless steel fibers, aluminum fibers, and brass fibers, organic fibers such as polyester fibers and aromatic polyamide fibers, gypsum fibers, ceramic fibers, asbestos fibers, zirconia fibers, alumina fibers, silica fibers, titanium oxide fibers, silicon carbide fibers, rock wool, potassium titanate whiskers, silicon nitride whiskers, fibrous or whisker-like filling materials of wollastonite, alumina silicate, and the like, and glass fibers, carbon fibers, aromatic polyamide fibers, and polyester fibers coated with one or more metals selected from the group consisting of nickel, copper, cobalt, silver, aluminum, iron, and alloys thereof. Two or more of these may be contained. The content of the fibrous filling material is preferably 1 to 200 parts by mass with respect to 100 parts by mass of the waste (A) of the resin molded body.

In the waste (A) of the resin molded body of the present invention, a filler other than the fibrous filling material, a thermoplastic resin other than polyamide 6, various additives, or the like can be further blended as long as the object of the present invention is not impaired. The filler other than the fibrous filling material may be either an organic filler or an inorganic filler, and examples thereof include non-fibrous filling materials, and two or more of these may be blended. Examples of the non-fibrous filling material include non-swellable silicates such as talc, wollastonite, zeolite, sericite, mica, kaolin, clay, pyrophyllite, bentonite, asbestos, alumina silicate, and calcium silicate, swellable layered silicates such as Li-type fluoroteniolite, Na-type fluoroteniolite, Na-type tetrasilicon fluorine mica, and Li-type tetrasilicon fluorine mica, metal oxides such as silicon oxide, magnesium oxide, alumina, silica, diatomaceous earth, zirconium oxide, titanium oxide, iron oxide, zinc oxide, calcium oxide, tin oxide, and antimony oxide, metal carbonates such as calcium carbonate, magnesium carbonate, zinc carbonate, barium carbonate, dolomite, and hydrotalcite, metal sulfates such as calcium sulfate and barium sulfate, metal hydroxides such as magnesium hydroxide, calcium hydroxide, aluminum hydroxide, and basic magnesium carbonate, smectite-based clay minerals such as montmorillonite, beidellite, nontronite, saponite, hectorite, and sauconite, various clay minerals such as vermiculite, halloysite, kanemite, kenyaite, zirconium phosphate, and titanium phosphate, glass beads, glass flakes, ceramic beads, boron nitride, aluminum nitride, silicon carbide, calcium phosphate, carbon black, and graphite. In the swellable layered silicate described above, an exchangeable cation present between layers may be exchanged with an organic onium ion. Examples of the organic onium ion include an ammonium ion, a phosphonium ion, and a sulfonium ion.

Specific examples of the various additives include a heat stabilizer such as a phenolic compound such as N,N'-hexamethylenebis(3,5-di-t-butyl-4-hydroxyhydrocinnamide) or tetrakis[methylene-3-(3',5'-di-t-butyl-4'-hydroxyphenyl)propionate]methane, a phosphorus-based compound, a sulfur-based compound such as a mercaptobenzimidazole-based compound, a dithiocarbamic acid-based compound, or an organic thioacid-based compound, or an amine-based compound such as N,N'-di-2-naphthyl-p-phenylenediamine or 4,4'-bis(α,α-dimethylbenzyl)diphenylamine, a coupling agent such as an isocyanate-based compound, an organic silane-based compound, an organic titanate-based compound, an organic borane-based compound, or an epoxy compound, a plasticizer such as a polyalkylene oxide oligomer-based compound, a thioether-based compound, an ester-based compound, or an organic phosphorus-based compound, a crystal nucleating agent such as an organic phosphorus compound or polyether ether ketone, a metal soap such as a montanic acid wax, lithium stearate, or aluminum stearate, a release agent such as ethylenediamine-stearic acid-sebacic acid polycondensate or a silicone-based compound, an anti-coloring agent such as a hypophosphite, a lubricant, an ultraviolet light inhibitor, a colorant, a flame retardant, and a foaming agent. When such an additive is contained, the content thereof is preferably 10 parts by mass or less, and more preferably 1 part by mass or less with respect to 100 parts by mass of polyamide 6.

Specific examples of the thermoplastic resin other than polyamide 6 contained in the waste (A) of the resin molded body include a polyamide resin other than polyamide 6, a polyester resin, a polyolefin resin, a modified polyphenylene ether resin, a polysulfone resin, a polyketone resin, a polyetherimide resin, a polyarylate resin, a polyethersulfone resin, a polyetherketone resin, a polythioetherketone resin, a polyetheretherketone resin, a polyimide resin, a polyamideimide resin, a tetrafluorinated polyethylene resin, and a polyphenylene sulfide resin. Two or more of these may be blended. Further, it can be preferably exemplified that the blending amount of the thermoplastic resin other than polyamide 6 here is set to 30 parts by mass or less with respect to 100 parts by mass of polyamide 6 in the waste (A) of the resin molded body of the present invention.

### (2) Thermoplastic resin

The present invention is a method for producing a thermoplastic resin, in which ε-caprolactam is obtained using a waste (A) of a resin molded body containing at least polyamide 6 as a raw material; and a raw material containing the ε-caprolactam is polymerized.

The thermoplastic resin here may be any thermoplastic resin obtained by polymerizing a raw material containing ε-caprolactam, and may be, for example, polyamide 6 in which ε-caprolactam is used as a main raw material, or a material in which ε-caprolactam is used as a main raw material and a monomer other than ε-caprolactam is copolymerized, or a block copolymer with a polyalkylene glycol such as polyethylene glycol. Examples of the monomer other than ε-caprolactam include amino acids such as 11-aminoundecanoic acid, 12-aminododecanoic acid, and para-aminomethylbenzoic acid, lactams such as ω-laurolactam, aliphatic diamines such as tetramethylenediamine, pentamethylenediamine, hexamethylenediamine, 2-methylpentamethylenediamine, nonamethylenediamine, decamethylenediamine, undecamethylenediamine, dodecamethylenediamine, 2,2,4-/2,4,4-trimethylhexamethylenediamine, and 5-methylnonamethylenediamine, aromatic diamines such as meta-xylylenediamine and para-xylylenediamine, alicyclic diamines such as 1,3-bis(aminomethyl)cyclohexane, 1,4-bis(aminomethyl)cyclohexane, 1-amino-3-aminomethyl-3,5,5-trimethylcyclohexane, bis(4-aminocyclohexyl)methane, bis(3-methyl-4-aminocyclohexyl)methane, 2,2-bis(4-aminocyclohexyl)propane, 1,4-bis(3-aminopropyl)piperazine, and 1-(2-aminoethyl)piperazine, aliphatic dicarboxylic acids such as adipic acid, suberic acid, azelaic acid, sebacic acid, and dodecanedioic acid, aromatic dicarboxylic acids such as terephthalic acid, isophthalic acid, 2-chloroterephthalic acid, 2-methylterephthalic acid, 5-methylisophthalic acid, 5-sodium sulfoisophthalate, and 2,6-naphthalenedicarboxylic acid, and alicyclic dicarboxylic acids such as 1,4-cyclohexanedicarboxylic acid, 1,3-cyclohexanedicarboxylic acid, 1,2-cyclohexanedicarboxylic acid, and 1,3-cyclopentanedicarboxylic acid. Two or more of these may be copolymerized.

The thermoplastic resin preferably contains a total of 50 mol% or more, more preferably 70 mol% or more, still more preferably 90 mol% or more of a unit derived from ε-caprolactam in a total of 100 mol% of monomer units included in the thermoplastic resin.

Since the present invention relates to a method for depolymerizing a waste of a resin molded body containing at least polyamide 6, the thermoplastic resin is preferably polyamide 6 from the viewpoint of repeated circulation utilization of fossil resources.

Further, the polymerization degree of the thermoplastic resin of the present invention is not particularly limited, but the relative viscosity measured at 25°C in a 98% concentrated sulfuric acid solution having a resin concentration of 0.01 g/mL is preferably in a range of 1.5 to 5.0. When the relative viscosity of the thermoplastic resin is in such a preferable range, there is a tendency that strength, stiffness, and toughness, which are characteristics of a thermoplastic resin obtained using ε-caprolactam as a main raw material, can all be achieved, and therefore the range can be mentioned as a preferable range.

### (3) Polyamide 6 oligomer

The polyamide 6 oligomer used in the present invention is a polyamide 6 oligomer in which 6-aminocaproic acid and/or ε-caprolactam is contained as a main component. Another monomer may be contained as long as the object of the present invention is not impaired. The phrase "contained as a main component" here means that a total of 50 mol% or more of a unit derived from 6-aminocaproic acid or a unit derived from ε-caprolactam is contained in a total of 100 mol% of monomer units included in the polyamide 6 oligomer. A unit derived from 6-aminocaproic acid or a unit derived from ε-caprolactam is more preferably contained in an amount of 70 mol% or more, and still more preferably 90 mol% or more.

Examples of another monomer contained in the polyamide 6 oligomer include amino acids such as 11-aminoundecanoic acid, 12-aminododecanoic acid, and para-aminomethylbenzoic acid, lactams such as ω-laurolactam, aliphatic diamines such as tetramethylenediamine, pentamethylenediamine, hexamethylenediamine, 2-methylpentamethylenediamine, nonamethylenediamine, decamethylenediamine, undecamethylenediamine, dodecamethylenediamine, 2,2,4-/2,4,4-trimethylhexamethylenediamine, and 5-methylnonamethylenediamine, aromatic diamines such as meta-xylylenediamine and para-xylylenediamine, alicyclic diamines such as 1,3-bis(aminomethyl)cyclohexane, 1,4-bis(aminomethyl)cyclohexane, 1-amino-3-aminomethyl-3,5,5-trimethylcyclohexane, bis(4-aminocyclohexyl)methane, bis(3-methyl-4-aminocyclohexyl)methane, 2,2-bis(4-aminocyclohexyl)propane, bis(aminopropyl)piperazine, aminoethylpiperazine aliphatic dicarboxylic acids such as adipic acid, suberic acid, azelaic acid, sebacic acid, and dodecanedioic acid, aromatic dicarboxylic acids such as terephthalic acid, isophthalic acid, 2-chloroterephthalic acid, 2-methylterephthalic acid, 5-methylisophthalic acid, 5-sodium sulfoisophthalate, 2,6-naphthalenedicarboxylic acid, hexahydroterephthalic acid, and hexahydroisophthalic acid, and alicyclic dicarboxylic acids such as 1,4-cyclohexanedicarboxylic acid, 1,3-cyclohexanedicarboxylic acid, 1,2-cyclohexanedicarboxylic acid, and 1,3-cyclopentanedicarboxylic acid. Two or more of these may be contained.

The number average molecular weight of the polyamide 6 oligomer of the present invention is not particularly limited, but it can be exemplified that the number average molecular weight is preferably in a range of 100 to 5,000, more preferably in a range of 200 to 3,000, and particularly preferably in a range of 200 to 2,000. When the molecular weight of the polyamide 6 oligomer is in such a preferable range, the solubility in water is increased, and a polyamide 6 oligomer aqueous solution (B1) used in the present invention tends to be easily prepared. The number average molecular weight here was calculated by GPC analysis using 1,1,1,3,3,3-hexafluoro-2-propanol as a solvent. GPC-HFIP-805 manufactured by Showa Denko K.K. was used as a column, and PMMA was used as a standard substance.

Further, the composition of the polyamide 6 oligomer used in the present invention is not particularly limited, but it can be preferably exemplified that the content of a 2- to 12-mer linear polyamide 6 oligomer contained in the polyamide 6 oligomer is 90% mass% or more. It can be exemplified that the content of a linear 2- to 12-mer oligomer is more preferably 93 mass% or more, and particularly preferably 95 mass% or more. When the content of a 2- to 12-mer linear polyamide 6 oligomer contained in the polyamide 6 oligomer is in such a preferable range, the solubility in water is increased, and further the concentration of the terminal carboxylic acid in the polyamide 6 oligomer is increased, so that the reaction of polyamide 6 with water is promoted, and the production efficiency of ε-caprolactam tends to increase. The amount of a linear 2- to 12-mer oligomer in the polyamide 6 oligomer here was quantitatively analyzed by high-performance liquid chromatography using a formic acid aqueous solution and a formic acid acetonitrile solution as eluents.

The method for preparing the polyamide 6 oligomer used in the present invention is not particularly limited, and for example, a polyamide 6 oligomer contained in an extract liquid when a polyamide 6 resin is subjected to hot water extraction at the time of production of a general fatty acid-based polyamide 6 resin, or a polyamide 6 oligomer prepared by the same method as a method for synthesizing a general fatty acid-based polyamide 6 resin may be used. In addition, a polyamide 6 oligomer obtained as a by-product when ε-caprolactam is produced by adding a resin composition containing at least polyamide 6 and at least one of water heated to 290°C or higher and 350°C or lower and a polyamide 6 oligomer aqueous solution heated to 290°C or higher and 350°C or lower may be used. Further, a polyamide 6 oligomer obtained as a by-product when ε-caprolactam is produced by using a waste (A) of a resin molded body containing polyamide 6 as a resin composition containing polyamide 6, and adding at least one of water (B) heated to 290°C or higher and 350°C or lower and a polyamide 6 oligomer aqueous solution (B1) heated to 290°C or higher and 350°C or lower thereto to make contact with each other may be used. From the viewpoint of reducing an industrial waste in production of ε-caprolactam, it is preferable to use a polyamide 6 oligomer obtained as a by-product when ε-caprolactam is produced by bringing a waste (A) of a resin molded body containing polyamide 6 into contact with water (B) heated to 290°C or higher and 350°C or lower, or a polyamide 6 oligomer collected as a by-product when ε-caprolactam is produced by adding a waste (A) of a resin molded body containing at least polyamide 6 and at least one of water heated to 290°C or higher and 350°C or lower and a polyamide 6 oligomer aqueous solution heated to 290°C or higher and 350°C or lower.

### (4) Polyamide 6 oligomer aqueous solution (B1)

The polyamide 6 oligomer aqueous solution (B1) used in the present invention is prepared by heating and mixing the polyamide 6 oligomer and water.

The water used when the polyamide 6 oligomer aqueous solution (B1) is prepared is not particularly limited, and tap water, ion exchanged water, distilled water, well water, or the like can be used, but ion exchanged water or distilled water is preferably used from the viewpoint of preventing side reactions due to the influence of coexisting salts.

In addition, the concentration of the polyamide 6 oligomer in the polyamide 6 oligomer aqueous solution (B1) used in the present invention may be any concentration as long as the polyamide 6 oligomer is dissolved in water when heated to 290°C or higher and 350°C or lower, but can be preferably exemplified by 20 mass% or less, more preferably 15 mass% or less, and still more preferably 10 mass% or less. When the concentration of the polyamide 6 oligomer is in such a preferable range, solubility in water when the polyamide 6 oligomer aqueous solution (B1) is prepared is increased, and the polyamide 6 oligomer aqueous solution (B1) can be prepared at a lower temperature.

Further, the extract liquid containing a polyamide 6 oligomer obtained in the step of extracting a polyamide 6 oligomer with hot water from polyamide 6, which is a product at the time of production of polyamide 6, can also be used as the polyamide 6 oligomer aqueous solution (B1). Usually, a polyamide 6 resin obtained by polymerizing ε-caprolactam contains unreacted monomers generated in a polymerization equilibrium reaction and polyamide 6 oligomers as impurities. Therefore, in order to remove them, a pellet after polymerization is supplied to a hot water extraction tower, and unreacted monomers and polyamide 6 oligomers are extracted and removed by hot water extraction. Use of the extract liquid obtained in the step of extracting a polyamide 6 oligomer with hot water from polyamide 6 at the time of production of polyamide 6 as the polyamide 6 oligomer aqueous solution (B1) of the present invention can also be exemplified as a preferable form from the viewpoint of industrial waste reduction.

### (5) Non-melting material

The non-melting material in the present invention may be any non-melting material contained in the waste (A) of the resin molded body containing at least polyamide 6. Specifically, the non-melting material is a material present in a solid state in the reaction mixture when the waste (A) of the resin molded body is depolymerized by adding at least one of water (B) heated to 290°C or higher and 350°C or lower and a polyamide 6 oligomer aqueous solution (B1) heated to 290°C or higher and 350°C or lower. More specific examples thereof include fillers such as a fibrous filling material, a non-fibrous filling material, and an elastomer used in production of a resin molded body containing polyamide 6, and a metal part and a rubber part contained in the waste (A) of the resin molded body containing polyamide 6. When polyamide 6 is depolymerized by adding at least one of water (B) heated to 290°C or higher and 350°C or lower and a polyamide 6 oligomer aqueous solution (B1) heated to 290°C or higher and 350°C or lower, these non-melting materials remain in a solid state in the reaction mixture, and thus can be separated from ε-caprolactam and the polyamide 6 oligomer by solid-liquid separation.

### (6) Step (a)

The method for producing a thermoplastic resin composition of the present invention includes a step (a) of adding at least one of water (B) heated to 290°C or higher and 350°C or lower and a polyamide 6 oligomer aqueous solution (B1) heated to 290°C or higher and 350°C or lower to the waste (A) of the resin molded body to make contact with each other.

The water (B) or the water in the polyamide 6 oligomer aqueous solution (B1) used here is not particularly limited, and tap water, ion exchanged water, distilled water, well water, or the like can be used, but ion exchanged water or distilled water is preferably used from the viewpoint of preventing side reactions due to the influence of coexisting salts.

In the step (a), the water (B) heated to 290°C or higher and 350°C or lower or the water in the polyamide 6 oligomer aqueous solution (B1) heated to 290°C or higher and 350°C or lower serves as a reaction substrate. When the pressure is increased to 22.1 MPa and the temperature is increased to 374.2°C, water is in a state of being not a liquid or a gas. This point is referred to as a critical point of water, and hot water having a temperature and a pressure lower than the critical point is referred to as subcritical water. The water used in the present invention is subcritical water, and the polyamide 6 oligomer aqueous solution is subcritical water in which the polyamide 6 oligomer is dissolved. Although the subcritical water is water, the subcritical water has characteristics that (i) the permittivity is low and (ii) the ion product is high, and the permittivity and the ion product of the subcritical water depend on the temperature and the partial pressure of water and can be controlled. Due to its low permittivity, the subcritical water serves as an excellent solvent for an organic compound even though it is water, and due to its high ion product, the hydrogen ion and hydroxide ion concentrations are increased, so that the subcritical water has an excellent hydrolysis action. The temperature of the water (B) and the polyamide 6 oligomer aqueous solution (B1) of the present invention is preferably 300°C or higher and 340°C or lower, and more preferably 320°C or higher and 340°C or lower. When the temperature is in such a preferable range, there is a tendency that device corrosion during reaction can be prevented. Further, the pressure of water when at least one of the water (B) and the polyamide 6 oligomer aqueous solution (B1) is added can be preferably exemplified by a pressure higher than the saturated vapor pressure. As the water, water in a liquid state or water in a gas state such as water vapor or both may be used, but the pressure of water is preferably higher than the saturated vapor pressure because the reaction is more likely to proceed in a liquid state than in a gas state in a reaction field. Further, the upper limit of the pressure of water is not particularly limited, but can be exemplified by 20 MPa or less. Such a pressure range is preferable because the ion product of the water tends to increase. In order to bring the water into such a pressure range, a method of pressurizing the inside of a pressure vessel and sealing the pressure vessel can be mentioned. In order to pressurize the inside of the pressure vessel, a gas may be enclosed in addition to the water (B) or the polyamide 6 oligomer aqueous solution (B1), and examples of such a gas include air, argon, and nitrogen, but it is preferable to use nitrogen or argon from the viewpoint of preventing side reactions such as an oxidation reaction. The degree of gas pressurization is not particularly limited because it is set to achieve a target pressure, but may be 0.3 MPa or more.

Further, the amount of water used in the water (B) and the polyamide 6 oligomer aqueous solution (B1) to be used is not particularly limited, but it can be preferably exemplified that the amount of water used is adjusted so that the product of X and Y satisfies the condition of 2,000 or less when the mass ratio of the water (B) to polyamide 6 in the waste (A) of the resin molded body, the mass ratio of the sum of the water (B) and water in the polyamide 6 oligomer aqueous solution (B1) to the sum of polyamide 6 and the polyamide 6 oligomer, or the mass ratio of water in the polyamide 6 oligomer aqueous solution (B1) to the sum of polyamide 6 and the polyamide 6 oligomer is represented by X : 1, and the reaction temperature is represented by Y°C. It can be exemplified that the product of X and Y preferably satisfies the condition of 1,600 or less, more preferably satisfies the condition of 1,300 or less, and particularly preferably satisfies the condition of 1,200 or less. Further, the lower limit of the product of X and Y is not particularly limited, but can be exemplified by the condition of preferably 300 or more, more preferably 320 or more, and particularly preferably 340 or more. The present invention relates to a method in which ε-caprolactam is collected in an energy saving manner from a waste of a resin molded body containing polyamide 6, and a thermoplastic resin is produced from the ε-caprolactam aiming at achieving both circulation utilization of fossil resources and reduction in global warming gas emissions. Since water has a specific heat capacity of 4.3 kJ/kg·K and a vaporization heat of 2,250 kJ/kg, which are very high as compared with other organic solvents, it is important to reduce the amount of water used, and when the product of X and Y is in such a preferable range, it is possible to achieve both production efficiency of ε-caprolactam and energy saving.

Further, when the retention time at the reaction temperature Y°C is represented by Z minutes, the product of X, Y, and Z preferably satisfies the condition of 60,000 or less. The product of X, Y, and Z more preferably satisfies the condition of 40,000 or less, still more preferably 30,000 or less, and particularly preferably 20,000 or less. Further, the lower limit of the product of X, Y, and Z is not particularly limited, but can be exemplified by the condition of preferably 5,000 or more, more preferably 8,000 or more, and particularly preferably 9,000 or more. The product of X, Y, and Z in such a preferable condition range is preferable because the production efficiency of ε-caprolactam and a thermoplastic resin in an energy saving manner tends to increase. In the reaction of polyamide 6 with water, in addition to the production of ε-caprolactam, a side reaction of linear oligomer production by the reaction of ε-caprolactam with water proceeds as a side reaction, and when the amount of water used is simply reduced, a large amount of linear oligomers are produced, and therefore the production efficiency of ε-caprolactam is greatly reduced. As a result of elucidating the thermodynamic equilibrium point of the production reaction of ε-caprolactam by the reaction of polyamide 6 with water and the side reaction of linear oligomer production, the present inventors have found that by setting the product of X and Y and the product of X, Y, and Z within the above ranges, by-production of linear oligomers is prevented, and the production efficiency of ε-caprolactam is greatly improved, leading to the present invention.

In addition, as a method of the reaction in which the waste (A) of the resin molded body and at least one of the water (B) and the polyamide 6 oligomer aqueous solution (B1) are added to make contact with each other, various known reaction methods such as a batch method and a continuous method can be adopted. Examples of the batch method include an autoclave and a vertical/horizontal reactor, both having a stirrer and a heating function, and a vertical/horizontal reactor having a compression mechanism such as a cylinder in addition to a stirrer and a heating function. Examples of the continuous method include an extruder and a tubular reactor, both having a heating function, a tubular reactor having a mixing mechanism such as a baffle, a line mixer, a vertical/horizontal reactor, a vertical/horizontal reactor having a stirrer, and a tower. The atmosphere in the production is desirably a non-oxidizing atmosphere, preferably an inert atmosphere of nitrogen, helium, argon, or the like, and is preferably a nitrogen atmosphere from the viewpoint of economy and ease of handling.

### (7) Step (b)

In the present invention, a step (b) of separating the reaction mixture containing the polyamide 6 oligomer and an ε-caprolactam aqueous solution obtained in the step (a) into a solid and the ε-caprolactam aqueous solution by solid-liquid separation (I) is included. Here, the solid contains the non-melting material and the polyamide 6 oligomer precipitated.

The temperature at which the solid-liquid separation (I) is performed may be in any temperature range as long as the solid and the ε-caprolactam aqueous solution can be separated, but can be exemplified by a temperature range not higher than the boiling point of water at the operation pressure, and is preferably a temperature not higher than the boiling point of water at normal pressure. When the waste (A) of the resin molded body contains a non-melting material, it can be exemplified that the solid-liquid separation (I) is more preferably performed at a temperature of 95°C or lower, and still more preferably performed at 90°C or lower. When the waste (A) of the resin molded body does not contain a non-melting material, it can be exemplified that the temperature at which the solid-liquid separation (I) is performed is more preferably 80°C or lower, still more preferably 60°C or lower, and particularly preferably 50°C or lower. Further, the lower limit temperature at which the solid-liquid separation (I) is performed is not particularly limited, but is preferably 10°C or higher, more preferably 15°C or higher, and still more preferably 20°C or higher. When the waste (A) of the resin molded body contains a non-melting material, most of the non-melting material can be separated as a solid component by performing the solid-liquid separation (I) at a temperature at which the non-melting material exists as a solid and the polyamide 6 oligomer is dissolved in water. When the waste (A) of the resin molded body does not contain a non-melting material, ε-caprolactam is dissolved in water in the above-mentioned temperature range, but the polyamide 6 oligomer tends to be less soluble in water. Therefore, by performing the solid-liquid separation (I) in the above-mentioned preferable temperature range, most of the polyamide 6 oligomer can be separated as a solid component, and ε-caprolactam with high purity can be collected.

The method for performing the solid-liquid separation (I) is not particularly limited, and a known method can be adopted, and pressure filtration or vacuum filtration, which is filtration using a filter, centrifugation or precipitation separation, which is separation based on a difference in specific gravity between a solid component and a solution, a combination thereof, or the like can be adopted. A decanter separation method in which precipitation separation is performed before a filtration operation is also a preferable method. The filter used for the filtration operation may be any filter as long as it is stable under the conditions for performing the solid-liquid separation (I), and for example, a filter sieve or a sintered plate can be suitably used. In addition, the mesh diameter or pore diameter of the filter can be adjusted in a wide range depending on the viscosity, pressure, and temperature of the reaction mixture to be subjected to the filtration operation, the size of the non-melting material, the purity (solid component content) of the filtrate to be obtained, and the like. In particular, it is effective to select the mesh diameter or the pore diameter according to the size of the non-melting material to be collected as a solid phase component by the solid-liquid separation (I) in the reaction mixture.

In addition, it is necessary to select a filter medium that can separate the polyamide 6 oligomer and allow a solution containing at least ε-caprolactam and water to pass therethrough as a filter medium used when the solid-liquid separation (I) is performed to obtain the polyamide 6 oligomer as a filter residue. Usually, a sieve having a hole diameter smaller than 200 mesh (mesh opening: 0.074 mm), or a filter medium having a pore diameter in a range of 70 µm to 0.01 µm, preferably in a range of 40 µm to 0.05 µm, more preferably in a range of 20 µm to 0.1 µm, still more preferably in a range of 5 µm to 0.1 µm, and yet still more preferably in a range of 1 µm to 0.1 µm can be exemplified. When a filter medium having a pore diameter in the above-mentioned range is used, the amount of the polyamide 6 oligomer passing through the filter medium tends to decrease, and the purity of ε-caprolactam when water is removed from the filtrate and ε-caprolactam is collected tends to increase. In addition, the collection rate of the polyamide 6 oligomer collected by the solid-liquid separation (I) tends to increase, and therefore, such a filter medium is preferable. Meanwhile, when the pore diameter is less than or equal to the above-mentioned preferable range, the filtration efficiency tends to deteriorate. Examples of a filter device include, but are not limited to, a method using a filter device such as a sieve, a method using a centrifugal separator, a method using a centrifugal filter device, a method using a vibration screen, a method using a pressure filter device, and a method using a suction filter device.

It is preferable that a mother liquor adhering to the solid component separated into the solid and the liquid by the solid-liquid separation (I) of the present invention is washed with water heated to the temperature at which the solid-liquid separation (I) is performed, so that the mother liquor substantially does not adhere thereto.

The atmosphere in which the solid-liquid separation (I) is performed is not particularly limited, but when the polyamide 6 oligomer separated as a solid component is oxidatively deteriorated depending on conditions such as time and temperature when contact is made, the solid-liquid separation (I) is preferably performed in a non-oxidizing atmosphere. Incidentally, the non-oxidizing atmosphere refers to an atmosphere in which the oxygen concentration in the gas phase is 5 vol% or less, preferably 2 vol% or less, and more preferably an atmosphere substantially containing no oxygen, that is, an inert gas atmosphere of nitrogen, helium, argon, or the like, and among these, it is particularly preferable to perform the solid-liquid separation (I) in a nitrogen atmosphere from the viewpoint of economy and ease of handling.

Examples of the solid-liquid separation (I) of the present invention include a method in which solid-liquid separation is performed by reheating the reaction mixture separately obtained in the step (a) to a temperature at which the solid-liquid separation (I) is performed, and a method in which the solid-liquid separation (I) is performed by cooling the reaction mixture to a temperature at which the solid-liquid separation (I) is performed after the step (a). Preferably, a method in which the solid-liquid separation (I) is performed by cooling to a temperature at which the solid-liquid separation (I) is performed after the step (a) can be mentioned.

When the solid-liquid separation (I) is performed, a seed crystal may be added to facilitate precipitation of the polyamide 6 oligomer. The seed crystal can be added at the start of cooling or during cooling. Since the seed crystal used here is preferably a crystal of the same substance as the polyamide 6 oligomer, it is preferable to use polyamide 6 or the polyamide 6 oligomer as the seed crystal. The use of such a seed crystal promotes the precipitation of the polyamide 6 oligomer, and the purity of ε-caprolactam collected by the solid-liquid separation (I) tends to increase.

Further, the polyamide 6 oligomer in the form of a wet cake collected as a solid phase component by the solid-liquid separation (I) contains moisture, but can be used in a wet cake state as a raw material of the polyamide 6 oligomer aqueous solution (B1) without being subjected to a drying treatment. Raw material utilization of the polyamide 6 oligomer collected by the solid-liquid separation (I) without being subjected to a drying step in this manner is preferable because the amount of industrial waste is reduced, and the energy required for a drying step can be omitted.

When the waste (A) of the resin molded body containing polyamide 6 used in the present invention contains a non-melting material, it is preferable to perform a step (b1) of separating the reaction mixture obtained in the step (a) into a non-melting material and an aqueous solution containing at least ε-caprolactam and a polyamide 6 oligomer by solid-liquid separation, and a step (b2) of separating a filtrate obtained in the step (b1) into the polyamide 6 oligomer and an ε-caprolactam aqueous solution by solid-liquid separation. The solid-liquid separation of the non-melting material in the step (b1) is preferable because the amount of the polyamide 6 oligomer to be subjected to industrial waste disposal together with the non-melting material is reduced, and further, the raw material utilization of the polyamide 6 oligomer is facilitated.

The temperature at which the step (b1) is performed may be in any temperature range as long as the non-melting material can be separated into a solid phase and the polyamide 6 oligomer and ε-caprolactam can be separated into a liquid phase. Since the depolymerization reaction of the waste (A) of the resin molded body is performed at 290°C or higher and 350°C or lower in the step (a), the temperature is preferably not higher than these temperatures. Further, as a result of studying the dissolution behavior and precipitation behavior of the polyamide 6 oligomer in water in the present invention, the present inventors have found that the temperature at which the polyamide 6 oligomer is dissolved in water is 100°C or higher, whereas the temperature at which the polyamide 6 oligomer once melted and dissolved in water is precipitated is lower than 100°C. The reason why the difference between the temperature at which the polyamide 6 oligomer is dissolved in water and the temperature at which the polyamide 6 oligomer is precipitated occurs in this manner that the polyamide 6 oligomer is in a supercooled state in a temperature range lower than 100°C and not lower than the temperature at which the polyamide 6 oligomer is precipitated. In general, in order to precipitate the dissolved polyamide 6 oligomer, the process goes through a stage in which first, a crystal nucleus of the polyamide 6 oligomer is formed, and the formed crystal nucleus grows to a sufficient size for precipitation to precipitate the polyamide 6 oligomer. Therefore, it is considered that, when the polyamide 6 oligomer aqueous solution dissolved in water is cooled, a crystal nucleus of the polyamide 6 oligomer is formed at a stage of cooling to lower than 100°C, which is a temperature at which the polyamide 6 oligomer is dissolved in water, and then the crystal nucleus formed in the cooling process grows to precipitate the polyamide 6 oligomer. From the above results, it can be exemplified that the step (b1) is more preferably performed in a temperature range not higher than the boiling point at normal pressure, still more preferably at 95°C or lower, and yet still more preferably at 90°C or lower. Further, the lower limit temperature at which the step (b1) is performed may be a temperature at which the polyamide 6 oligomer is in a supercooled state, and can be specifically exemplified by preferably 50°C or higher, more preferably 60°C or higher, and still more preferably 70°C or higher. When the step (b1) is performed in such a preferable temperature range, 97% or more, preferably 98% or more, more preferably 99% or more of ε-caprolactam or the polyamide 6 oligomer is transformed into a collectable state as a liquid phase component, and there is a tendency that the collection loss in the step (b1) can be reduced. Further, examples of the step (b1) of the present invention include a method in which the reaction mixture prepared in the step (a) is reheated to a temperature at which the polyamide 6 oligomer is dissolved and cooled to a temperature at which the step (b1) is performed to perform solid-liquid separation, and a method in which the reaction mixture is prepared in the step (a) and then cooled to a temperature at which the step (b1) is performed to perform solid-liquid separation, and preferable examples thereof include a method in which the reaction mixture is prepared in the step (a) and then cooled to a temperature at which the step (b1) is performed to perform solid-liquid separation.

According to the solid-liquid separation in the step (b1), the non-melting material in the reaction mixture obtained in the step (a) can be separated as a filter residue, and preferably 95% or more, more preferably 97% or more, and still more preferably 99% or more of the non-melting material contained in the mixture can be collected as a solid component. In addition, when an aqueous solution containing ε-caprolactam and the polyamide 6 oligomer adheres to the non-melting material which is the filter residue separated in the step (b1), the amount of the ε-caprolactam and the polyamide 6 oligomer remaining in the filter residue can be reduced by washing the filter residue with fresh water. The washing of the filter residue with fresh water here is performed in a temperature range not higher than the boiling point at normal pressure, and a temperature range of preferably 95°C or lower, more preferably 90°C or lower can be mentioned. The separation of the non-melting material from ε-caprolactam and the polyamide 6 oligomer by solid-liquid separation and washing with water in the step (b1) in this manner eliminates the need for a complicated process, and thus can be said to be a preferable method also from the viewpoint of process cost or environmental load.

### (8) Method for collecting ε-caprolactam

The method for collecting ε-caprolactam from the filtrate obtained by the solid-liquid separation (I) of the present invention is not particularly limited, and any method can be adopted. For example, ε-caprolactam with high purity can be collected by performing a distillation operation of the ε-caprolactam aqueous solution obtained by the solid-liquid separation (I) to separate ε-caprolactam from water and the polyamide 6 oligomer. In addition, if a water-insoluble component is precipitated by cooling the ε-caprolactam aqueous solution collected by the solid-liquid separation (I), the water-insoluble component is separated in advance by a known method such as solid-liquid separation and can also be subjected to distillation separation.

Further, as a method for obtaining ε-caprolactam with high purity, it can be combined with a purification method such as a method for precisely distilling collected ε-caprolactam, a vacuum distillation method by adding a trace amount of sodium hydroxide, an activated carbon treatment method, an ion exchange treatment method, or a recrystallization method. By these methods, impurities that are difficult to separate by distillation separation can also be efficiently removed.

### (9) Method for producing thermoplastic resin

The present invention relates to a method for producing a thermoplastic resin by obtaining ε-caprolactam with high purity from a waste (A) of a resin molded body containing polyamide 6 and polymerizing a raw material containing the ε-caprolactam. The thermoplastic resin can be produced by a generally known method, and examples thereof include a method of subjecting ε-caprolactam to hot melt polymerization in the presence of a small amount of water, a method of subjecting ε-caprolactam and a copolymerization component to hot melt polymerization in the presence of a small amount of water, and a method of subjecting ε-caprolactam and a block copolymer component such as polyethylene glycol to hot melt polymerization in the presence of a small amount of water.

Further, the thermoplastic resin thus obtained is melt-kneaded with a fibrous filling material or various additives as necessary to produce a thermoplastic resin composition, and various molded products such as a sheet and a film can be obtained by a generally known method such as injection molding or extrusion molding.

The thermoplastic resin of the present invention and a molded product thereof can be used for various applications such as electrical/electronic parts, building members, various vessels, daily necessities, household goods, and sanitary goods by taking advantage of its excellent properties. In particular, it is particularly preferably used for aircraft parts and electrical/electronic parts which require toughness and stiffness. Specifically, aircraft-related parts such as a landing gear pod, a winglet, a spoiler, an edge, a ladder, an elevator, a fairing, and a rib, and as electrical/electronic parts, for example, electrical parts such as an electrical generator, an electric motor, a transformer, a current transformer, a voltage regulator, a rectifier, a resistor, an inverter, a relay, a power contact, an electrical switch, an interrupter, a switch, a knife switch, a multi-pole rod, a motor case, a TV housing, a laptop housing and internal parts, a CRT display housing and internal parts, a printer housing and internal parts, mobile terminal housings and internal parts such as a mobile phone, a mobile personal computer, and a handheld mobile terminal, IC- and LED-compatible housings, a capacitor plate, a fuse holder, various gears, various cases, and a cabinet, and electronic parts such as a connector, an SMT-compatible connector, a card connector, a jack, a coil, a coil bobbin, a sensor, an LED lamp, a socket, a resistor, a relay, a relay case, a reflector, a small switch, a power supply part, a coil bobbin, a capacitor, a variable capacitor case, an optical pickup chassis, an oscillator, various terminal boards, a transformer, a plug, a printed circuit board, a tuner, a speaker, a microphone, a headphone, a small motor, a magnetic head base, a power module, a Si power module and a SiC power module, a semiconductor, a liquid crystal, a FDD carriage, a FDD chassis, a motor brush holder, a transformer member, a parabolic antenna, and a computer-related part, and the like are mentioned.

### [Examples]

Hereinafter, the present invention will be described with reference to examples, but the present invention is not limited by these examples.

In each example, the following raw materials were used.
· Used fastener part made of non-reinforced polyamide 6 (content proportion of polyamide 6 in resin component: 99 mass% or more)
· Used mobile phone housing made of glass fiber-reinforced polyamide 6 (the content proportion of polyamide 6 in the resin component is 99 mass% or more, and the proportion of glass fibers in the resin composition is 45 mass%)

### <<Evaluation Method>>

### [Yield of ε-caprolactam (HPLC)]

The calculation of the yield of ε-caprolactam (HPLC) of the present invention was performed by high-performance liquid chromatography measurement. The measurement conditions are as follows.
Apparatus: LC-10Avp series manufactured by Shimadzu Corporation
Column: Mightysil RP-18GP 150-4.6
Detector: Photodiode array detector (UV = 205 nm)
Flow rate: 1 mL/min
Column temperature: 40°C
Mobile phase: 0.1% acetic acid aqueous solution/acetonitrile
Sample: A high-performance liquid chromatography analysis sample was prepared by taking about 0.1 g of the reaction mixture, diluting it with about 10 g of deionized water, and separating and removing components insoluble in deionized water by filtration.
Quantification of ε-caprolactam: The amount of ε-caprolactam with respect to polyamide 6 was quantified by an absolute calibration curve method.

### [Analysis of polyamide 6 oligomer (HPLC)]

Analysis of the polyamide 6 oligomer of the present invention was performed by high-performance liquid chromatography measurement. The measurement conditions are as follows.
Apparatus: LC-10Avp series manufactured by Shimadzu Corporation
Column: Mightysil RP-18GP 150-4.6
Detector: Photodiode array detector (UV = 205 nm)
Flow rate: 1 mL/min
Column temperature: 40°C
Mobile phase: 0.1% formic acid aqueous solution/0.1% formic acid acetonitrile solution
Polyamide 6 oligomer composition: the amount of a linear 2- to 12-mer oligomer in the polyamide 6 oligomer is calculated from the peak area ratio of each polyamide 6 oligomer

### [Thermal property analysis of polyamide 6]

The melting point of polyamide 6 here was defined as the temperature of an endothermic peak appearing when the polyamide was cooled from a molten state to 30°C at a cooling rate of 20°C/min and then heated to the melting point + 40°C at a heating rate of 20°C/min in a nitrogen gas atmosphere using differential scanning calorimeter analysis. However, when two or more endothermic peaks were detected, the temperature of the endothermic peak having a highest peak intensity was defined as the melting point.

### [Measurement of solution viscosity of polyamide 6]

Here, the solution viscosity ηr of polyamide 6 was measured at 25°C using a 0.01 g/mL solution of 98% concentrated sulfuric acid.

### [Reference Example 1] Hot water extraction of polyamide 6

In a 70-liter autoclave, 20 kg of ε-caprolactam, 4.32 g of benzoic acid, and 3.0 kg of ion exchanged water were charged, the inside of the polymerization can was sealed and sufficiently purged with nitrogen, and then the temperature was raised until the pressure inside the can of the polymerization reactor reached 0.98 MPa with stirring, and the temperature was continuously raised to 250°C while the pressure inside the can was maintained. After the temperature reached 250°C, the pressure was released to atmospheric pressure over 40 minutes. Thereafter, the mixture was held at 250°C for 180 minutes at atmospheric pressure, and then the polyamide 6 polymer was discharged and cooled/cut to form a pellet.

This pellet was subjected to extraction using 20 times the amount of hot water at 98°C to collect an extract liquid containing unreacted caprolactam and a polyamide 6 oligomer. The total amount of the unreacted caprolactam and the polyamide 6 oligomer in the extract liquid was 0.5 mass%, and the amount of the polyamide 6 oligomer was 0.1 mass%.

### [Example 1]

A used fastener part made of non-reinforced polyamide 6 (the content proportion of polyamide 6 was 99 mass% or more) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 20.0 g of the crushed product and 60.0 g of deionized water were charged. Since the content proportion of polyamide 6 in the fastener part is 99 mass% or more, the mass ratio (X : 1) of water to polyamide 6 is 3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. The ultimate pressure during the reaction was 10.5 MPa. Since the reaction temperature Y°C is 320°C, the product of X and Y is 960, and since the retention time at the reaction temperature of 320°C is 15 minutes, the product of X, Y, and Z is 14,400.

After completion of the reaction, the internal temperature was cooled to 50°C, the bottom plug valve was opened while the temperature was maintained at 50°C to perform solid-liquid separation (I). The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 50°C 3 times, and the filtrate and the wet filter residue were collected.

The filter residue obtained by the solid-liquid separation (I) was subjected to vacuum drying at 50°C for 12 hours to collect 2.7 g of a solid component. The obtained solid component was subjected to high-performance liquid chromatography analysis under the conditions described above and found to be a polyamide 6 oligomer containing 96.5 mass% of a linear 2- to 12-mer oligomer. This polyamide 6 oligomer has high purity, and therefore can be further utilized as a depolymerization raw material.

Further, as a result of high-performance liquid chromatography measurement of the filtrate obtained by the solid-liquid separation (I), the amount of ε-caprolactam contained in the filtrate was 15.0 g, and the yield with respect to the polyamide 6 in the crushed product used as the raw material was 75.2%.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was collected from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.69.

### [Example 2]

A used mobile phone housing made of glass fiber-reinforced polyamide 6 (the content proportion of polyamide 6 in the resin is 99 mass% or more, and the content proportion of glass fibers in the resin composition is 45 mass%) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 36.4 g of the crushed product and 60.0 g of deionized water were charged. Since the content proportion of polyamide 6 in the resin component in the mobile phone housing is 99 mass% or more, and the content proportion of glass fibers in the resin composition is 45 mass%, the mass ratio of water to polyamide 6 (X : 1) is 3 : 1.

The reaction vessel was purged with nitrogen and sealed under a nitrogen pressure of 0.5 MPa, and then the reaction was carried out by holding the reaction vessel at 340°C for 15 minutes with stirring at 200 rpm. Since the reaction temperature Y°C is 340°C, the product of X and Y is 1,020, and since the retention time at the reaction temperature of 340°C is 15 minutes, the product of X, Y, and Z is 15,300.

After completion of the reaction, the internal temperature was cooled to 50°C, and the bottom plug valve was opened while the temperature was maintained at 50°C to perform solid-liquid separation (I) of a non-melting material. The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 50°C 3 times, and the filtrate and the wet filter residue were collected.

As a result of high-performance liquid chromatography measurement of the filtrate obtained by the solid-liquid separation (I), the amount of ε-caprolactam contained in the filtrate was 14.4 g, and the yield with respect to the polyamide 6 in the crushed product used as the raw material was 71.9%.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was collected from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.69.

### [Example 3]

A used mobile phone housing made of glass fiber-reinforced polyamide 6 (the content proportion of polyamide 6 in the resin is 99 mass% or more, and the content proportion of glass fibers in the resin composition is 45 mass%) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 36.4 g of the crushed product and 60.0 g of deionized water were charged. Since the content proportion of polyamide 6 in the resin component in the mobile phone housing is 99 mass% or more, and the content proportion of glass fibers in the resin composition is 45 mass%, the mass ratio of water to polyamide 6 (X : 1) is 3 : 1.

The reaction vessel was purged with nitrogen and sealed under a nitrogen pressure of 0.5 MPa, and then the reaction was carried out by holding the reaction vessel at 340°C for 15 minutes with stirring at 200 rpm. Since the reaction temperature Y°C is 340°C, the product of X and Y is 1,020, and since the retention time at the reaction temperature of 340°C is 15 minutes, the product of X, Y, and Z is 15,300.

After completion of the reaction, the internal temperature was cooled to 90°C, the bottom plug valve was opened while the temperature was maintained at 90°C, and glass fibers as a non-melting material were separated by solid-liquid separation (step (b1)). The solid-liquid separation was performed while nitrogen was introduced into the autoclave at 0.3 MPa at the stage when the filtration speed decreased. Further, the filter residue was washed by rinsing using about 3 times the amount (mass) of deionized water heated to 90°C to the filter residue, and the filtrate and the wet filter residue were collected.

Further, the filtrate obtained in the step (b1) was cooled to an internal temperature of 25°C, and further subjected to solid-liquid separation using a glass filter having an average opening of 10 to 16 µm (step (b2)). Further, the filter residue was washed by rinsing 3 times using about 3 times the amount of deionized water heated to 25°C to the filter residue, and the filtrate and the wet filter residue were collected.

As a result of high-performance liquid chromatography measurement of the filtrate obtained in the step (b2), the amount of ε-caprolactam contained in the filtrate was 14.1 g, and the yield with respect to the polyamide 6 in the crushed product used as the raw material was 70.5%.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was collected from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.69.

In addition, the wet filter residue obtained in the step (b1) was subjected to vacuum drying at 50°C for 12 hours to collect 16.2 g of glass fibers. In a crucible, 1.0 g of the collected glass fibers were weighed and treated in an air atmosphere for 3 hours in an electric furnace heated to 600°C, and the amount of organic substances adhered to the collected glass fibers was evaluated from the mass loss, and as a result, it was found that the mass loss was 1.3 mass%, and the collected glass fibers were glass fibers with high purity having a small amount of organic substances adhered thereto.

In addition, the wet filter residue obtained in the step (b2) was subjected to vacuum drying at 50°C for 12 hours to collect 2.0 g of a solid component by solid-liquid separation (I). The obtained solid component was subjected to high-performance liquid chromatography analysis under the conditions described above and found to be a polyamide 6 oligomer containing 97.6 mass% of a linear 2- to 12-mer oligomer. This polyamide 6 oligomer has high purity, and therefore can be further utilized as a depolymerization raw material.

### [Example 4]

A used mobile phone housing made of glass fiber-reinforced polyamide 6 (the content proportion of polyamide 6 in the resin is 99 mass% or more, and the content proportion of glass fibers in the resin composition is 45 mass%) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 32.7 g of the crushed product and 27.8 g of deionized water were weighed, and 34.5 g of a polyamide 6 oligomer aqueous solution having a concentration of 5.8 mass% prepared using a polyamide 6 oligomer collected by the method described in Example 3 was further added thereto. Since the content proportion of polyamide 6 in the resin component in the mobile phone housing is 99 mass% or more, and the content proportion of glass fibers in the resin composition is 45 mass%, the mass ratio of water to the sum of polyamide 6 and the polyamide 6 oligomer (X : 1) is 3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. Since the reaction temperature Y°C is 320°C, the product of X and Y is 960, and since the retention time at the reaction temperature of 320°C is 15 minutes, the product of X, Y, and Z is 14,400.

After completion of the reaction, the internal temperature was cooled to 90°C, the bottom plug valve was opened while the temperature was maintained at 90°C to perform solid-liquid separation (step (b1)). The solid-liquid separation was performed while nitrogen was introduced into the autoclave at 0.3 MPa at the stage when the filtration speed decreased. Further, the filter residue was washed by rinsing using about 3 times the amount (mass) of deionized water heated to 90°C to the filter residue, and the filtrate and the wet filter residue were collected.

Further, the filtrate obtained in the step (b1) was cooled to an internal temperature of 30°C, and subjected to solid-liquid separation using a glass filter having an average opening of 10 to 16 µm (step (b2)). Further, the filter residue was washed by rinsing 3 times using about 3 times the amount of deionized water heated to 30°C to the filter residue, and the filtrate and the wet filter residue were collected.

As a result of high-performance liquid chromatography measurement of the filtrate collected by the step (b2), the amount of ε-caprolactam contained in the filtrate was 13.8 g, and the yield with respect to the polyamide 6 in the crushed product used as the raw material was 76.7%. Comparison with Example 3 shows that the yield of ε-caprolactam with respect to polyamide 6 is increased by further using a polyamide 6 oligomer aqueous solution when the reaction of polyamide 6 with water is carried out.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was taken out from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.70.

In addition, the wet filter residue obtained in the step (b1) was subjected to vacuum drying at 50°C for 12 hours to collect 14.7 g of glass fibers. In a crucible, 1.0 g of the collected glass fibers were weighed and treated in an air atmosphere for 3 hours in an electric furnace heated to 600°C, and the amount of organic substances adhered to the collected glass fibers was evaluated from the mass loss, and as a result, it was found that the mass loss was 1.4 mass%, and the collected glass fibers were glass fibers with high purity having a small amount of organic substances adhered thereto.

In addition, the wet filter residue obtained in the step (b2) was subjected to vacuum drying at 50°C for 12 hours to collect 2.0 g of a solid component by solid-liquid separation (I). The obtained solid component was subjected to high-performance liquid chromatography analysis under the conditions described above and found to be a polyamide 6 oligomer containing 97.8 mass% of a linear 2- to 12-mer oligomer. This polyamide 6 oligomer has high purity, and therefore can be further utilized as a depolymerization raw material.

### [Comparative Example 1]

A used fastener part made of non-reinforced polyamide 6 (the content proportion of polyamide 6 was 99 mass% or more) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, 20.0 g of the crushed product and 60.0 g of deionized water were charged. Since the content proportion of polyamide 6 in the fastener part is 99 mass% or more, the mass ratio (X : 1) of water to polyamide 6 is 3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm, followed by cooling to collect the reaction mixture. Since the reaction temperature Y°C is 320°C, the product of X and Y is 960, and since the retention time at the reaction temperature of 320°C is 15 minutes, the product of X, Y, and Z is 14,400.

As a result of high-performance liquid chromatography measurement of the obtained reaction mixture, the amount of ε-caprolactam was 15.0 g, and the yield with respect to the polyamide 6 in the crushed product used as the raw material was 75.2%.

Further, the reaction mixture was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. Meanwhile, as a result of analysis of the distillation residue, a component insoluble in a solvent was generated due to long-time heating at a high temperature during distillation, and further utilization was difficult, and thus the distillation residue was subjected to industrial waste disposal.

Comparison between Example 1 and Comparative Example 1 shows that the present method is excellent as a method for performing circulation utilization of fossil resources because a polyamide component to be subjected to industrial waste disposal among the raw material polyamide 6 components can be significantly reduced by performing the solid-liquid separation (I).

### [Example 5]

A used fastener part made of non-reinforced polyamide 6 (the content proportion of polyamide 6 was 99 mass% or more) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 45.0 g of the crushed product and 135.0 g of deionized water were charged. Since the content proportion of polyamide 6 in the fastener part is 99 mass% or more, the mass ratio (X : 1) of water to polyamide 6 is 3 : 1.

The reaction vessel was purged with nitrogen and sealed under a nitrogen pressure of 5.0 MPa, and then the reaction was carried out by holding the reaction vessel at 320°C for 15 minutes with stirring at 200 rpm. The ultimate pressure during the reaction was 19.6 MPa. Further, since the reaction temperature Y°C is 320°C, the product of X and Y is 960, and since the retention time at the reaction temperature of 320°C is 15 minutes, the product of X, Y, and Z is 14,400.

After completion of the reaction, the internal temperature was cooled to 50°C, the bottom plug valve was opened while the temperature was maintained at 50°C to perform solid-liquid separation (I). The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 50°C 3 times, and the filtrate and the wet filter residue were collected.

The filter residue obtained by the solid separation (I) was subjected to vacuum drying at 50°C for 12 hours to collect 4.8 g of a solid component. The obtained solid component was subjected to high-performance liquid chromatography analysis under the conditions described above and found to be a polyamide 6 oligomer containing 97.1 mass% of a linear 2- to 12-mer oligomer. This polyamide 6 oligomer has high purity, and therefore can be further utilized as a depolymerization raw material.

Further, as a result of high-performance liquid chromatography measurement of the filtrate obtained by the solid-liquid separation (I), the amount of ε-caprolactam contained in the filtrate was 37.4 g, and the yield with respect to the polyamide 6 in the crushed product used as the raw material was 83.0%.

Comparison with Example 1 shows that the yield of the obtained ε-caprolactam tends to be improved by setting the pressure during the reaction to a high pressure equal to or higher than the saturated vapor pressure.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10.0 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was collected from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.69.

### [Example 6]

A used mobile phone housing made of glass fiber-reinforced polyamide 6 (the content proportion of polyamide 6 in the resin is 99 mass% or more, and the content proportion of glass fibers in the resin composition is 45 mass%) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 68.0 g of the crushed product and 112.0 g of deionized water were charged. Since the content proportion of polyamide 6 in the resin component in the mobile phone housing is 99 mass% or more, and the content proportion of glass fibers in the resin composition is 45 mass%, the mass ratio of water to polyamide 6 (X : 1) is 3 : 1.

The reaction vessel was purged with nitrogen and sealed under a nitrogen pressure of 5.0 MPa, and then the reaction was carried out by holding the reaction vessel at 320°C for 15 minutes with stirring at 200 rpm. The ultimate pressure during the reaction was 19.8 MPa. Further, since the reaction temperature Y°C is 320°C, the product of X and Y is 960, and since the retention time at the reaction temperature of 320°C is 15 minutes, the product of X, Y, and Z is 14,400.

After completion of the reaction, the internal temperature was cooled to 90°C, the bottom plug valve was opened while the temperature was maintained at 90°C, and glass fibers as a non-melting material were separated by solid-liquid separation (step (b1)). The solid-liquid separation was performed while nitrogen was introduced into the autoclave at 0.3 MPa at the stage when the filtration speed decreased. Further, the filter residue was washed by rinsing using about 3 times the amount (mass) of deionized water heated to 90°C to the filter residue, and the filtrate and the wet filter residue were collected.

Further, the filtrate obtained in the step (b1) was cooled to an internal temperature of 25°C, and further subjected to solid-liquid separation using a glass filter having an average opening of 10 to 16 µm (step (b2)). Further, the filter residue was washed by rinsing 3 times using about 3 times the amount of deionized water heated to 25°C to the filter residue, and the filtrate and the wet filter residue were collected.

As a result of high-performance liquid chromatography measurement of the filtrate obtained in the step (b2), the amount of ε-caprolactam contained in the filtrate was 29.9 g, and the yield with respect to the polyamide 6 in the crushed product used as the raw material was 80.0%.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10.0 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was collected from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.69.

In addition, the wet filter residue obtained in the step (b1) was subjected to vacuum drying at 50°C for 12 hours to collect 30.6 g of glass fibers. In a crucible, 1.0 g of the collected glass fibers were weighed and treated in an air atmosphere for 3 hours in an electric furnace heated to 600°C, and the amount of organic substances adhered to the collected glass fibers was evaluated from the mass loss, and as a result, it was found that the mass loss was 1.2 mass%, and the collected glass fibers were glass fibers with high purity having a small amount of organic substances adhered thereto.

In addition, the wet filter residue obtained in the step (b2) was subjected to vacuum drying at 50°C for 12 hours to collect 3.6 g of a solid component by solid-liquid separation (I). The obtained solid component was subjected to high-performance liquid chromatography analysis under the conditions described above and found to be a polyamide 6 oligomer containing 97.4 mass% of a linear 2- to 12-mer oligomer. This polyamide 6 oligomer has high purity, and therefore can be further utilized as a depolymerization raw material.

### [Example 7]

A used fastener part made of non-reinforced polyamide 6 (the content proportion of polyamide 6 was 99 mass% or more) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 30.0 g of the crushed product and 60.0 g of deionized water were charged. Since the content proportion of polyamide 6 in the fastener part is 99 mass% or more, the mass ratio (X : 1) of water to polyamide 6 is 2 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. Since the reaction temperature Y°C is 320°C, the product of X and Y is 640, and since the retention time at the reaction temperature of 320°C is 15 minutes, the product of X, Y, and Z is 9,600.

After completion of the reaction, the internal temperature was cooled to 50°C, the bottom plug valve was opened while the temperature was maintained at 50°C to perform solid-liquid separation (I). The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 50°C 3 times, and the filtrate and the wet filter residue were collected.

The filter residue obtained by the solid-liquid separation (I) was subjected to vacuum drying at 50°C for 12 hours to collect 6.5 g of a solid component. The obtained solid component was subjected to high-performance liquid chromatography analysis under the conditions described above and found to be a polyamide 6 oligomer containing 97.5 mass% of a linear 2- to 12-mer oligomer. This polyamide 6 oligomer has high purity, and therefore can be further utilized as a depolymerization raw material.

Further, as a result of high-performance liquid chromatography measurement of the filtrate obtained by the solid-liquid separation (I), the amount of ε-caprolactam contained in the filtrate was 19.5 g, and the yield with respect to the polyamide 6 in the crushed product used as the raw material was 65.0%.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10.0 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was collected from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.69.

### [Example 8]

A used fastener part made of non-reinforced polyamide 6 (the content proportion of polyamide 6 was 99 mass% or more) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 60.0 g of the crushed product and 120.0 g of deionized water were charged. Since the content proportion of polyamide 6 in the fastener part is 99 mass% or more, the mass ratio (X : 1) of water to polyamide 6 is 2 : 1.

The reaction vessel was purged with nitrogen and sealed under a nitrogen pressure of 5.0 MPa, and then the reaction was carried out by holding the reaction vessel at 320°C for 15 minutes with stirring at 200 rpm. The ultimate pressure during the reaction was 19.6 MPa. Further, since the reaction temperature Y°C is 320°C, the product of X and Y is 640, and since the retention time at the reaction temperature of 320°C is 15 minutes, the product of X, Y, and Z is 9,600.

After completion of the reaction, the internal temperature was cooled to 50°C, the bottom plug valve was opened while the temperature was maintained at 50°C to perform solid-liquid separation (I). The solid-liquid separation (I) was performed while nitrogen was introduced into the autoclave at 0.3 MPa at the stage when the filtration speed decreased. Further, about 3 times the amount (mass) of deionized water to the filter residue was introduced into the autoclave, and the filter residue was washed by rinsing at 50°C 3 times, and the filtrate and the wet filter residue were collected.

The filter residue obtained by the solid-liquid separation (I) was subjected to vacuum drying at 50°C for 12 hours to collect 9.2 g of a solid component. The obtained solid component was subjected to high-performance liquid chromatography analysis under the conditions described above and found to be a polyamide 6 oligomer containing 97.1 mass% of a linear 2- to 12-mer oligomer. This polyamide 6 oligomer has high purity, and therefore can be further utilized as a depolymerization raw material.

Further, as a result of high-performance liquid chromatography measurement of the filtrate obtained by the solid-liquid separation (I), the amount of ε-caprolactam contained in the filtrate was 45.0 g, and the yield with respect to the polyamide 6 in the crushed product used as the raw material was 75.0%. Comparison with Example 7 shows that the yield of the obtained ε-caprolactam tends to be improved by setting the pressure during the reaction to a high pressure equal to or higher than the saturated vapor pressure.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10.0 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was collected from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.69.

### [Example 9]

A used mobile phone housing made of glass fiber-reinforced polyamide 6 (the content proportion of polyamide 6 in the resin is 99 mass% or more, and the content proportion of glass fibers in the resin composition is 45 mass%) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 60.0 g of the crushed product and 48.9 g of deionized water were weighed, and 67.7 g of a polyamide 6 oligomer aqueous solution having a concentration of 6.5 mass% prepared using a polyamide 6 oligomer collected by the method described in Example 3 was further added thereto. Since the content proportion of polyamide 6 in the resin component in the mobile phone housing is 99 mass% or more, and the content proportion of glass fibers in the resin composition is 45 mass%, the mass ratio of water to the sum of polyamide 6 and the polyamide 6 oligomer (X : 1) is 3 : 1.

The reaction vessel was purged with nitrogen and sealed under a nitrogen pressure of 5.0 MPa, and then the reaction was carried out by holding the reaction vessel at 320°C for 15 minutes with stirring at 200 rpm. At this time, the ultimate pressure during the reaction was 19.6 MPa. Further, since the reaction temperature Y°C is 320°C, the product of X and Y is 960, and since the retention time at the reaction temperature of 320°C is 15 minutes, the product of X, Y, and Z is 14,400.

After completion of the reaction, the internal temperature was cooled to 90°C, the bottom plug valve was opened while the temperature was maintained at 90°C to perform solid-liquid separation (step (b1)). The solid-liquid separation was performed while nitrogen was introduced into the autoclave at 0.3 MPa at the stage when the filtration speed decreased. Further, the filter residue was washed by rinsing using about 3 times the amount (mass) of deionized water heated to 90°C to the filter residue, and the filtrate and the wet filter residue were collected.

Further, the filtrate obtained in the step (b1) was cooled to an internal temperature of 50°C, and subjected to solid-liquid separation using a glass filter having an average opening of 10 to 16 µm (step (b2)). Further, the filter residue was washed by rinsing 3 times using about 3 times the amount of deionized water heated to 50°C to the filter residue, and the filtrate and the wet filter residue were collected.

As a result of high-performance liquid chromatography measurement of the filtrate collected by the step (b2), the amount of ε-caprolactam contained in the filtrate was 27.1 g, and the yield with respect to the polyamide 6 in the crushed product used as the raw material was 82.0%. Comparison with Example 4 shows that the yield of the obtained ε-caprolactam tends to be improved by setting the pressure during the reaction to a high pressure equal to or higher than the saturated vapor pressure.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10.0 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was taken out from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.70.

In addition, the wet filter residue obtained in the step (b1) was subjected to vacuum drying at 50°C for 12 hours to collect 27.1 g of glass fibers. In a crucible, 1.0 g of the collected glass fibers were weighed and treated in an air atmosphere for 3 hours in an electric furnace heated to 600°C, and the amount of organic substances adhered to the collected glass fibers was evaluated from the mass loss, and as a result, it was found that the mass loss was 1.4 mass%, and the collected glass fibers were glass fibers with high purity having a small amount of organic substances adhered thereto.

In addition, the wet filter residue obtained in the step (b2) was subjected to vacuum drying at 50°C for 12 hours to collect 6.3 g of a solid component by solid-liquid separation (I). The obtained solid component was subjected to high-performance liquid chromatography analysis under the conditions described above and found to be a polyamide 6 oligomer containing 97.9 mass% of a linear 2- to 12-mer oligomer. This polyamide 6 oligomer has high purity, and therefore can be further utilized as a depolymerization raw material.

### [Example 10]

Here, an example, in which the hot water extract liquid in the PA6 production step obtained by the method described in Reference Example 1 was concentrated until the concentration of unreacted ε-caprolactam and the polyamide 6 oligomer reached 5.8 mass%, and used as a depolymerization raw material, is described.

A used mobile phone housing made of glass fiber-reinforced polyamide 6 (the content proportion of polyamide 6 in the resin is 99 mass% or more, and the content proportion of glass fibers in the resin composition is 45 mass%) was collected and put into a crusher having a screen with a diameter of 7 mm to obtain a crushed product with an average particle diameter of 6 mm, and visually recognizable contaminated foreign matter was removed.

In an autoclave made of SUSU316L equipped with a stirrer, a bottom plug valve, and a glass filter (average opening: 10 µm) at the bottom, 32.7 g of the crushed product and 27.8 g of deionized water were weighed, and 34.5 g of a concentrated liquid prepared by adjusting the concentration of the ε-caprolactam and the polyamide 6 oligomer to 5.8 mass% was further added. Since the content proportion of polyamide 6 in the resin component in the mobile phone housing is 99 mass% or more, and the content proportion of glass fibers in the resin composition is 45 mass%, the mass ratio of water to sum of polyamide 6 and the polyamide 6 oligomer (X : 1) is 3.3 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. Since the reaction temperature Y°C is 320°C, the product of X and Y is 1,050, and since the retention time at the reaction temperature of 320°C is 15 minutes, the product of X, Y, and Z is 15,740.

After completion of the reaction, the internal temperature was cooled to 90°C, the bottom plug valve was opened while the temperature was maintained at 90°C to perform solid-liquid separation (step (b1)). The solid-liquid separation was performed while nitrogen was introduced into the autoclave at 0.3 MPa at the stage when the filtration speed decreased. Further, the filter residue was washed by rinsing using about 3 times the amount (mass) of deionized water heated to 90°C to the filter residue, and the filtrate and the wet filter residue were collected.

Further, the filtrate obtained in the step (b1) was cooled to an internal temperature of 50°C, and subjected to solid-liquid separation using a glass filter having an average opening of 10 to 16 µm (step (b2)). Further, the filter residue was washed by rinsing 3 times using about 3 times the amount of deionized water heated to 50°C to the filter residue, and the filtrate and the wet filter residue were collected.

As a result of high-performance liquid chromatography measurement of the filtrate collected by the step (b2), the amount of ε-caprolactam contained in the filtrate was 13.6 g, and the yield with respect to the polyamide 6 in the crushed product used as the raw material was 75.6%. Comparison with Example 4 shows that the extract liquid in the polyamide 6 polymerization step can also be used as a polyamide 6 oligomer aqueous solution without any problem.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.8%. In addition, the HPLC impurity in the distilled ε-caprolactam was 0.48%, and the quality was such that it can be used also as a polymerization raw material of polyamide 6.

Subsequently, polymerization was performed using the obtained ε-caprolactam as a raw material. In a test tube, 10.0 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was taken out from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.70.

In addition, the wet filter residue obtained in the step (b1) was subjected to vacuum drying at 50°C for 12 hours to collect 14.7 g of glass fibers. In a crucible, 1.0 g of the collected glass fibers were weighed and treated in an air atmosphere for 3 hours in an electric furnace heated to 600°C, and the amount of organic substances adhered to the collected glass fibers was evaluated from the mass loss, and as a result, it was found that the mass loss was 1.3 mass%, and the collected glass fibers were glass fibers with high purity having a small amount of organic substances adhered thereto.

In addition, the wet filter residue obtained in the step (b2) was subjected to vacuum drying at 50°C for 12 hours to collect 1.8 g of a solid component by solid-liquid separation (I). The obtained solid component was subjected to high-performance liquid chromatography analysis under the conditions described above and found to be a polyamide 6 oligomer containing 97.4 mass% of a linear 2- to 12-mer oligomer. This polyamide 6 oligomer has high purity, and therefore can be further utilized as a depolymerization raw material.

### [Example 11]

Here, an example, in which the hot water extract liquid in the PA6 production step obtained by the method described in Reference Example 1 was concentrated until the total concentration of unreacted ε-caprolactam and the polyamide 6 oligomer reached 6.5 mass%, and used as a depolymerization raw material, is described.

A used mobile phone housing made of glass fiber-reinforced polyamide 6 (the content proportion of polyamide 6 in the resin is 99 mass% or more, and the content proportion of glass fibers in the resin composition is 45 mass%) was collected and put into a crusher having a screen with a diameter of 7 mm to obtain a crushed product with an average particle diameter of 6 mm, and visually recognizable contaminated foreign matter was removed.

In an autoclave made of SUS316L equipped with a stirrer, a bottom plug valve, and a glass filter (average opening: 10 µm) at the bottom, 36.0 g of the crushed product and 65.0 g of a concentrated liquid prepared by adjusting the concentration of the ε-caprolactam and the polyamide 6 oligomer to 6.5 mass% were weighed. Since the content proportion of polyamide 6 in the resin component in the mobile phone housing is 99 mass% or more, and the content proportion of glass fibers in the resin composition is 45 mass%, the mass ratio of water to the sum of polyamide 6 and the polyamide 6 oligomer (X : 1) is 2.9 : 1.

The reaction vessel was purged with nitrogen and sealed under a nitrogen pressure of 0.5 MPa, and then the reaction was carried out by holding the reaction vessel at 320°C for 15 minutes with stirring at 200 rpm. Since the reaction temperature Y°C is 320°C, the product of X and Y is 941, and since the retention time at the reaction temperature of 320°C is 15 minutes, the product of X, Y, and Z is 14,112.

After completion of the reaction, the internal temperature was cooled to 90°C, the bottom plug valve was opened while the temperature was maintained at 90°C to perform solid-liquid separation (step (b1)). The solid-liquid separation was performed while nitrogen was introduced into the autoclave at 0.3 MPa at the stage when the filtration speed decreased. Further, the filter residue was washed by rinsing using about 3 times the amount (mass) of deionized water heated to 90°C to the filter residue, and the filtrate and the wet filter residue were collected.

Further, the filtrate obtained in the step (b1) was cooled to an internal temperature of 50°C, and subjected to solid-liquid separation using a glass filter having an average opening of 10 to 16 µm (step (b2)). Further, the filter residue was washed by rinsing 3 times using about 3 times the amount of deionized water heated to 50°C to the filter residue, and the filtrate and the wet filter residue were collected.

As a result of high-performance liquid chromatography measurement of the filtrate collected by the step (b2), the amount of ε-caprolactam contained in the filtrate was 14.9 g, and the yield with respect to the polyamide 6 in the crushed product used as the raw material was 75.3%.

Further, the collected filtrate was heated to 55°C under a reduced pressure of 30 mmHg to separate water by distillation, thereby obtaining a concentrated ε-caprolactam aqueous solution, which was further distilled under a reduced pressure of 5 mmHg at a heating temperature of 150 to 170°C to collect distilled ε-caprolactam. The concentration and distillation yield of ε-caprolactam was 95.7%. In addition, the HPLC impurity in the distilled ε-caprolactam was 0.39%, and the quality was such that it can be used also as a polymerization raw material of polyamide 6.

Subsequently, polymerization was performed using the obtained ε-caprolactam as a raw material. In a test tube, 10.0 g of the collected ε-caprolactam, 2.2 mg of benzoic acid, and 10.0 g of ion exchanged water were weighed. The test tube was placed in an autoclave, the inside of the autoclave was purged with nitrogen, and then the jacket temperature was set to 250°C, and heating was started. After the internal pressure reached 1.0 MPa, the internal pressure was maintained at 1.0 MPa for 3 hours. Thereafter, the internal pressure was released to normal pressure over 1.5 hours, and heating was stopped when the internal temperature reached 228°C. After completion of the polymerization, the polymer was taken out from the test tube and subjected to a crushing treatment. The crushed polymer was treated in hot water at 95°C for 15 hours to extract and remove unreacted monomers and low polymers. The polymer after extraction was subjected to vacuum drying at 80°C for 24 hours to obtain a polyamide 6 resin having a melting point of 225°C and ηr = 2.70.

In addition, the wet filter residue obtained in the step (b1) was subjected to vacuum drying at 50°C for 12 hours to collect 16.3 g of glass fibers. In a crucible, 1.0 g of the collected glass fibers were weighed and treated in an air atmosphere for 3 hours in an electric furnace heated to 600°C, and the amount of organic substances adhered to the collected glass fibers was evaluated from the mass loss, and as a result, it was found that the mass loss was 1.3 mass%, and the collected glass fibers were glass fibers with high purity having a small amount of organic substances adhered thereto.

In addition, the wet filter residue obtained in the step (b2) was subjected to vacuum drying at 50°C for 12 hours to collect 1.7 g of a solid component by solid-liquid separation (I). The obtained solid component was subjected to high-performance liquid chromatography analysis under the conditions described above and found to be a polyamide 6 oligomer containing 97.9 mass% of a linear 2- to 12-mer oligomer. This polyamide 6 oligomer has high purity, and therefore can be further utilized as a depolymerization raw material.

## Claims

1. A method for producing a thermoplastic resin, comprising obtaining ε-caprolactam by steps (a) and (b) below using a waste (A) of a resin molded body containing at least polyamide 6 as a raw material, and polymerizing a raw material containing the ε-caprolactam:
(a) a step of adding at least one of water (B) heated to 290°C or higher and 350°C or lower and a polyamide 6 oligomer aqueous solution (B1) heated to 290°C or higher and 350°C or lower to the waste (A) of the resin molded body to make contact with each other; and
(b) a step of separating a reaction mixture obtained in the step (a) into a solid and an aqueous solution containing ε-caprolactam by a solid-liquid separation (I).

2. The method for producing a thermoplastic resin according to claim 1, wherein the step (a) is a step of adding water (B) heated to 290°C or higher and 350°C or lower, or adding water (B) heated to 290°C or higher and 350°C or lower and a polyamide 6 oligomer aqueous solution (B1) heated to 290°C or higher and 350°C or lower, to the waste (A) of the resin molded body to make contact with each other.

3. The method for producing a thermoplastic resin according to claim 1 or 2, wherein the solid-liquid separation (I) includes a step (b1) of separating the reaction mixture into a non-melting material and an aqueous solution containing at least ε-caprolactam and a polyamide 6 oligomer by a solid-liquid separation, and a step (b2) of separating a filtrate obtained in the step (b1) into the polyamide 6 oligomer and an ε-caprolactam aqueous solution by a solid-liquid separation.

4. The method for producing a thermoplastic resin according to any one of claims 1 to 3, wherein a polyamide 6 oligomer aqueous solution (B1) obtained by mixing the polyamide 6 oligomer separated in either the step (b) or the step (b2) with water, followed by heating to 290°C or higher and 350°C or lower is used in the step (a).

5. The method for producing a thermoplastic resin according to any one of claims 1 to 4, wherein the polyamide 6 oligomer aqueous solution (B1) is an extract liquid obtained in a step of extracting a polyamide 6 oligomer with hot water from polyamide 6 which is a product at a time of production of polyamide 6.

6. The method for producing a thermoplastic resin according to any one of claims 1 to 5, wherein in the step (a), the contact is made under a condition that a product of X and Y is 2,000 or less when a mass ratio of water to polyamide 6 in the waste (A) of the resin molded body or a mass ratio of water to a sum of polyamide 6 and a polyamide oligomer in the waste (A) of the resin molded body is represented by X : 1 and a reaction temperature is represented by Y°C.
